Europäisches Patentamt

European Patent Office

Office européen des brevets

(19)

(11) Publication number: 0 410 689 A1

# EUROPEAN PATENT APPLICATION

(21) Application number: 90308083.6

(22) Date of filing: 24.07.90

(51) Int. Cl.5: C10G 3/00, C10L 1/02

(30) Priority: 28.07.89 US 386178
16.10.89 US 422203

(43) Date of publication of application:
30.01.91 Bulletin 91/05

(84) Designated Contracting States:
BE DE FR GB IT NL

(71) Applicant: MOBIL OIL CORPORATION
3225 Gallows Road
Fairfax Virginia 22037(US)

(72) Inventor: Harandi, Mohsen N.
12 Catbird Court
Lawrenceville, New Jersey 08648(US)
Inventor: Owen, Hartley
5 Riverview
Belle Mead, New Jersey 08502(US)

(74) Representative: Curtis, Philip Anthony (GB)
Patent Department, Mobil Court 3 Clements
Inn
London WC2A 2EB(GB)

(54) Process for producing alkyl tertiary-alkyl ether and C5+ gasoline boiling range hydrocarbons.

(57) An integrated process is disclosed that substantially reduces the cost of producing MTBE and other alkyl tert-alkyl ethers by eliminating a major portion of the equipment and operating costs associated with the downstream processing of the etherification reactor effluent. The integrated process combines the process for the etherification of iso-olefins and methanol, or other alkanols, to produce methyl tertiary alkyl ether such as MTBE and/or TAME with the catalytic process for converting feedstock such as oxygenates, light olefins and paraffins to higher molecular weight hydrocarbons. Unconverted reactants from the etherification reaction, which may comprise unreacted alkanol and unreacted hydrocarbons or just unreacted hydrocarbons, are separated from the product ethers and passed to the catalytic conversion process reactor for conversion to gasoline boiling range hydrocarbons.

## PROCESS FOR PRODUCING ALKYL TERTIARY-ALKYL ETHERS AND C5+ GASOLINE BOILING RANGE HYDROCARBON

This invention relates to a process for producing alkyl tertiary-alkyl ethers and C5+ gasoline boiling range hydrocarbons. More specifically, the invention relates to methods for reacting aliphatic alcohol (alkanol), such as methanol or the like and olefinic hydrocarbons to produce lower alkyl tertiary-alkyl ethers without the need for recycling unreacted alkanol and/or hydrocarbon. In particular, this invention relates to a continuous integrated system for converting methanol to high octane ether and gasoline by etherifying hydrocarbons containing iso-olefins, such as the $C_3$-$C_4$ olefinic cracked gas streams obtained from fluid catalytic cracking (FCC) products.

It is known that isobutylene and other isoalkenes produced by hydrocarbon cracking may be reacted with methanol and other C1-C4 lower aliphatic alcohols over an acidic catalyst to provide methyl tertiary butyl ether (MTBE) or the like. Generally, it is known that asymmetrical ethers having the formula $(CH_3)_3$C-O-R, where R is a C1-C4 alkyl radical, are particularly useful as octane improvers for liquid fuels, especially gasoline.

MTBE, ethyl t-butyl ether (ETBE), tert-amyl methyl ether (TAME) and isopropyl t-butyl ether (IPTBE) are known to be high octane ethers. The article by J.D. Chase, et al., Oil and Gas Journal , April 9, 1979, discusses the advantages one can achieve by using such materials to enhance to enhance gasoline octane. The octane blending number of MTBE when 10% is added to a base fuel (R+O = 91) is about 120. For a fuel with a low motor rating (M+O = 83) octane, the blending value of MTBE at the 10% level is about 103. On the other hand, for an (R+O) of 95 octane fuel, the blending value of 10% MTBE is about 114.

While it is known that isobutylene and/or isoamylene may be reacted with methanol over an acidic catalyst to provide MTBE and/or TAME, a problem of major importance in these processes is the separation of unreacted methanol and hydrocarbons from the etherification reaction product due to the proclivity of methanol to form a very dilute azeotropic mixture with hydrocarbons and the strong solubility of methanol in both water and hydrocarbons. But, to achieve high iso-olefin conversion to ethers equimolar or larger quantities of methanol are preferred in the etherification reaction, producing unreacted methanol. Due largely to these factors, the cost associated with methanol separation and recycling in the etherification reaction represents a large part of the cost of the total etherification process.

In recent years, a major development within the petroleum industry has been the discovery of the special catalytic capabilities of a family of zeolite catalyst based upon medium size shape selective metallosilicates. Discoveries have been made leading to a series of analogous processes drawn from the catalytic capability of zeolites. Depending upon various conditions of space velocity, temperature and pressure lower oxygenates, such as methanol can be converted in the presence of zeolite type catalyst to olefins which can oligomerize to provide gasoline or distillate or can be converted further to produce aromatics. Recognizing the commonality of the feedstock and product between etherification reactions to produce high octane gasoline and zeolite catalyzed conversion reactions, interest has focused on the applicability of combined processes as an approach to advance the art in the production of high octane gasoline.

It has been discovered that under certain conditions substantial improvements in the art of alkyl tert-alkyl ether production can be realized in a combination or integration of etherification and hydrocarbon conversion processes based upon zeolite type catalysis. Accordingly, it is an object of this invention to provide an integrated process for the production alkyl tert-alkyl ethers, particularly MTBE and/or TAME.

Another object of this invention is to provide a cost effective process for the production of high octane alkyl tertiary alkyl ethers without the need to recycle unreacted alkanol and/ or unreacted hydrocarbons to the etherification reactor.

Yet another object of the present invention is to provide a process for the production of high octane gasoline rich in aromatics utilizing unreacted methanol from etherification.

According to one aspect of the present invention there is provided a continuous process for converting lower aliphatic alkanol and olefinic hydrocarbon to alkyl tertiary-alkyl ethers and $C_5$+ gasoline boiling range hydrocarbons comprising the steps of:

(a) contacting alkanol and a light olefinic hydrocarbon stream rich in $C_4$ isomeric hydrocarbons under iso-olefin etherification conditions in an etherification reaction zone containing acid etherification catalyst;

(b) separating etherification effluent from step (a) to recover a vapor stream comprising unreacted alkanol and light olefinic hydrocarbon and a liquid product stream containing alkyl tertiary-butyl ether; and

(c) contacting said unreacted alkanol and light olefinic hydrocarbon vapor stream with acidic, medium pore metallosilicate catalyst under alkanol and hydrocarbon conversion conditions whereby C5 + gasoline boiling range hydrocarbons are produced.

Preferably the molar ratio of said alkanol to iso-olefin is between substantially 0.7 and 2.0.

According to a further embodiment there is provided a continuous process for the production of aromatics-rich high octane gasoline, comprising; contacting a feedstream mixture containing a minor amount of lower alkanol and a major amount of C4- olefin in a reaction zone containing medium pore metallosilicate catalyst under aromatization conditions at elevated temperature; and; recovering a reaction zone effluent comprising aromatics-rich C5 + gasoline.

Reference is now made to the accompany drawing, which is a schematic etherification process flowsheet depicting the process according to the present invention.

Typical feedstock materials for etherification reactions include olefinic streams, such as FCC light cracked gas containing butene isomers, often in mixture with substantial amounts of propene, propane, n-butane and isobutane. The C4 components usually contain a major amounts of unsaturated compounds, such as 10-25% isobutylene, 25-55% linear butenes, and small amounts of butadiene. Also, C4 + heavier olefinic hydrocarbon streams may be used, particularly mixtures of isobutylene and isoamylene. These aliphatic streams are produced in a variety of petroleum refinery operations such as catalytic cracking of gas oil or the like. The dry methanol feedstream should preferably have a purity of about 99.8 wt%.

Suitable alkanols include C1-C4 primary or secondary alcohols, including methanol, ethanol, 1-propanol, isopropanol, 1-butanol, 2-butanol and mixtures thereof.

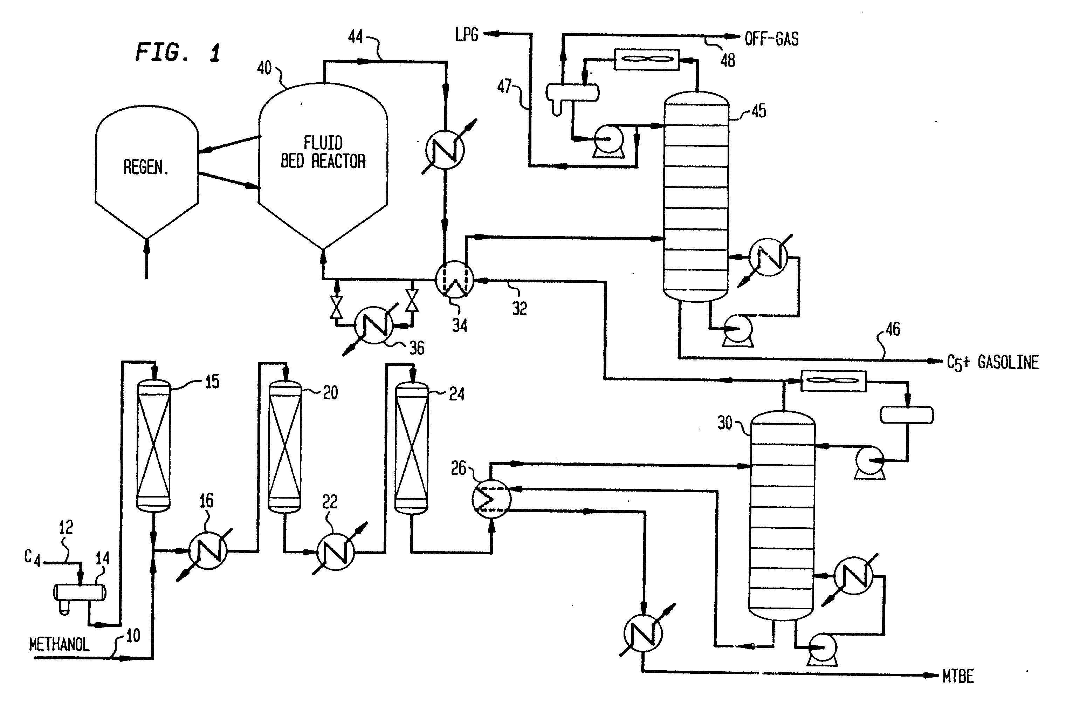

Referring to FIG. 1 of the drawing, a continuous stream of C4-olefinic hydrocarbon liquid is introduced via conduit 12 to pre-separation unit 14 and guard bed 15 and mixed with dry methanol (MeOH) feedstock introduced via conduit 10. The combined streams are pressurized to about 1190 kPa (170psig), heated in exchanger 16 to a temperature of about 55°C (130°F) and passed to a first serial reactor 20 for conversion of methanol in contact with acid etherification catalyst, preferably solid polysulfonic acid resin such as sulfonated vinyl aromatic resins. Amberlyst 15 from Rohm and Haas is a preferred catalyst. From first reactor 20 the reaction stream is cooled in exchanger 22 from about 70°C to 55°C and further reacted in a second serial reactor 24, where the reaction is carried partially to completion (e.g., 96% conversion of isobutylene).

The reactor effluent stream containing MTBE, methanol and unreacted light hydrocarbons is preheated in exchanger 26 and fed to debutanizer fractionation tower 30. In separation unit 30 the C5 + methyl tert-alkyl ether product is recovered as a liquid product, along with byproduct dimer or other heavier hydrocarbons in the effluent. Tower overhead vapor comprising unreacted C4- light hydrocarbons and methanol are passed via conduit 32 and heat exchange means 34, 36 to an effluent upgrading reactor 40. The catalytic conversion unit 40 is preferably a fluid bed unit, equipped with a catalyst regenerator 42. Reactor effluent may be fractionated in a debutanizer tower 45 to recover a gasoline product stream 46, C3-C4 LPG stream 47, and off-gas 48.

The methanol-containing stream, preferably a vapor stream, may be co-reacted with olefinic light gas and/or other reactive hydrocarbon feedstreams in an oligomerization/aromatization reaction section, as described by Avidan et al. in U.S. Patents 4,547,616 and 4,746,762 and by Owen et al. in U.S. 4,827,046 and U.S. 4,831,203. Optionally, FCC light gas containing ethene or propene may be injected at the bottom of the fluidized bed reaction zone and converted along with the unreacted methanol containing fractions of the light hydrocarbon etherification effluent stream.

The following Example provides a detailed illustration of the process of the invention for a refinery operation processing methanol and C4 feedstock to produce MTBE and hydrocarbon conversion products containing about 22 mole percent of gasoline products. The gasoline products are about 55 weight percent aromatics, 15 weight percent olefins, with the balance paraffins.

EXAMPLE

About 530.36 moles/hr of feedstock is passed to the etherification reactor system as described in Figure 1. The feedstock contains 69 moles/hr of methanol and hydrocarbons consisting of 12 moles/hr of C3's, 129.45 moles/hr of isobutane, 219.18 moles/hr of 1-butene, 25.09 moles/hr of n-butane and 71.18 moles/hr of isobutene and 4.46 moles/hr of C5 + hydrocarbons. Following etherification, the effluent therefrom contains 66.87 moles/hr of MTBE, 2.09 moles/hr of unreacted methanol, 4.27 moles/hr of unreacted isobutene, 12 moles/hr of C3's, 129.45 moles/hr of isobutane, 219.18 moles/hr of 1-butene, 25.09 moles/hr of n-butane and 4.46 moles/hr of C5 +. MTBE is recovered following debutanization and debutanized overhead vapor is passed to the fluidized bed zeolite conversion zone for contact with ZSM-5 catalyst under conversion conditions previously described with reference to Figure 1. The conversion is carried out at a temperature of 480°C (900°F) and 925kPa (135 psia) to produce 354.16 moles/hr of effluent. The product stream consists essentially of water (2.09 moles/hr), $C_5$ + gasoline (77.87 moles/hr), isobutene (6.63 moles/hr), n-butane (39.21 moles/hr), 1-butene (6.63 moles/hr), isobutane (153.71 moles/hr), propane (37.98 moles/hr), propene (14.32 moles/hr), ethane (3.84 moles/hr), ethene (8.68 moles/hr), and methane (3.20 moles/hr).

## Etherification Operation

The reaction of methanol with isobutylene and isoamylenes at moderate conditions with a resin catalyst is known technology, as provided by R. W. Reynolds, et al., The Oil and Gas Journal , June 16, 1975, and S. Pecci and T. Floris, Hydrocarbon Processing , December 1977. An article entitled "MTBE and TAME - A Good Octane Boosting Combo," by J.D. Chase, et al., The Oil and Gas Journal , April 9, 1979, pages 149-152, discusses the technology. A preferred catalyst is a polymeric sulfonic acid exchange resin such as Amberlyst 15.

In the etherification process it is known that alkanol and iso-olefins may be reacted in equimolar quantities or either reactant may be in molar excess to influence the complete conversion of the other reactant. Because etherification is an incomplete reaction the etherification effluent comprises unreacted alkanol and unreacted hydrocarbons. On a stoichiometric equivalencies basis, equimolar quantities of methanol and iso-olefins are advantageous but an excess between 2 and 200% of either component can be passed to the etherification reaction unit. In the present invention, the molar ratio of alkanol to iso-olefin, such as methanol to isobutylene, can be between 0.7 and 2, but preferably the molar ratio is 1 for methanol to isobutylene. Advantageously, the excess methanol may be about 40% or more when the hydrocarbon feedstream comprises significant quantity of isoamylenes, but equimolar quantities are preferred when the hydrocarbon feedstream consists essentially of $C_4$ hydrocarbons.

Iso-olefins or isoalkenes in this invention are those having the formula $R_2C = CH_2$ or $R_2C = CHR$. Alkanols which may be used in the present invention include methanol, ethanol, 1-propanol, isopropanol, 1-butanol and 2-butanol. In this invention oxygenate or lower oxygenates refers to $C_1$-$C_5$ alcohols, ethers, aldehydes, esters and the like.

## METHANOL AND/OR HYDROCARBONS CONVERSION

It is advantageous to convert substantially the entire stream of unreacted alcohol and light olefinic components recovered from etherification effluent by acid zeolite catalysis, thus providing a once-through process without expensive alcohol recycle to the etherification unit. Zeolite conversion technology for upgrading lower aliphatic hydrocarbons and oxygenates to liquid hydrocarbon products, including gasoline boiling range hydrocarbons and aromatics, are well known. Commercial Methanol-to-Gasoline (MTG), methanol-to olefins (MTO), Mobile Olefin to Gasoline/Distillate (MOG/D) and conversion of olefins and paraffins to aromatics (M-2 Forming) processes employ shape selective medium pore zeolite catalysts. It is understood that the present zeolite conversion unit operation can have the characteristics of these catalysts and processes to produce a variety of hydrocarbon products, especially liquid aliphatics and aromatics in the C5-C9 gasoline range. The zeolite catalyzed conversion processes can be run in fixed, fluidized or moving catalyst beds.

The various reactions which take place in the zeolite conversion reactor include oligomerization, alkylation, dehydrocyclization, isomerization and cracking. These include exothermic and endothermic reactions, which can be thermally balanced to require little or no heat exchange to maintain process reaction temperature in the fluidized bed. Mixed hydrocarbons from FCC operations, following the etherification and effluent separation, can be selected or modified to include a balance of olefins and paraffins to achieve the desired thermodynamic properties.

## Description of Zeolite Catalysts

Recent developments in zeolite technology have provided a group of medium pore siliceous materials having similar pore geometry. More prominent among these intermediate pore size zeolites is ZSM-5, which is usually synthesized with Bronsted acid active sites by incorporating a tetrahedrally coordinated metal, such as A1, Ga, Fe or mixtures thereof, within the zeolitic framework. These medium pore zeolites are favored for acid catalysis; however, the advantages of ZSM-5 structures may be utilized by employing highly siliceous materials or crystalline metallosilicate having one or more tetrahedral species having varying degrees of acidity. ZSM-5 crystalline structure is readily recognized by its X-ray diffraction pattern, which is described in U.S. Patent No. 3,702,866 (Argauer, et al.).

Zeolite hydrocarbon upgrading catalysts preferred for use herein include the medium pore (i.e., about 5-7A) shape-selective crystalline aluminosilicate zeolites having a silica-to-alumina ratio of at least 12, a constraint index of about 1 to 12 and acid cracking activity (alpha value) of about 1-250, preferably about 3 to 80 based on total catalyst weight. In the fluidized bed reactor the coked catalyst preferably has an apparent activity (alpha value) of about 1 to 20 under the process conditions to achieve the required degree of reaction severity. Representative of the ZSM-5 type medium pore shape selective zeolites are ZSM-5, ZSM-11, ZSM-12, ZSM-23, ZSM-35, and ZSM-48. Aluminosilicate ZSM-5 is disclosed in U.S. Patent No. 3,702,886 and U.S. Patent No. Re. 29,948. Other suitable zeolites are disclosed in U.S. Patents 3,709,979; 3,832,449; 4,076,979; 3,832,449; 4,076,842; 4,016,245; 4,414,423; 4,417,086; 4,517,396 and 4,542,251. While suitable zeolites having a coordinated metal oxide to silica molar ratio of 12:1 to 200:1 or higher may be used, it is advantageous to employ a standard ZSM-5 having a silica alumina molar ratio of about 25:1 to 70:1, suitably modified if desired to adjust acidity and oligomerization/aromatization characteristics. A typical zeolite catalyst component having Bronsted acid sites may consist essentially of aluminosilicate ZSM-5 zeolite.

Certain of the ZSM-5 type medium pore shape selective catalysts are sometimes known as pentasils. In addition to the preferred aminosilicates, the gallosilicate, the ferrosilicate materials may be employed. ZSM-5 type pentasil zeolites are particularly useful in the process because of their regenerability, long life and stability under the extreme conditions of operation. Usually the zeolite crystals have a crystal size from about 0.01 to 2 microns or more. In order to obtain the desired particle size for fluidization in the turbulent regime, the zeolite catalyst crystals are bound with a suitable inorganic oxide, such as silica, alumina, etc. to provide a zeolite concentration of about 5 to 95 wt.%. It is advantageous to employ a standard ZSM-5 having a silica:alumina molar ratio of 25:1 or greater in a once-through fluidized bed unit to convert 60 to 100 percent, preferably at least 75 wt%, of the monoalkenes and methanol in a single pass. In the preferred embodiment 25% H-ZSM-5 catalyst calcined with 75% silica-alumina matrix binder is employed unless otherwise stated.

Fluidized Bed Reactor Operation

Suitable olefinic feedstreams to the olefin upgrading unit comprise $C_2$-$C_4$ alkenes, including un-reacted butylenes and other alkenes from the etherification operation. Non-deleterious components, such as $C_4$ lower paraffins and inert gases, may be present. The reaction severity conditions can be controlled to optimize yield of olefinic gasoline or $C_6$-$C_8$ BTX hydrocarbons, according to product demand. Reaction temperatures and contact time are also significant factors in the reaction severity, and the process parameters are followed to give a substantially steady state condition wherein the reaction severity is maintained within the limits which yield a desired weight ratio of propane to propene in the reaction effluent.

In a turbulent fluidized catalyst bed the conversion reactions are conducted in a vertical reactor column by passing hot reactant vapor or lift gas upwardly through the reaction zone at a velocity greater than dense bed transition velocity and less than transport velocity for the average catalyst particle. A continuous process is operated by withdrawing a portion of coked catalyst from the reaction zone, oxidatively regenerating the withdrawn catalyst and returning regenerated catalyst to the reaction zone at a rate to control catalyst activity and reaction severity to effect feedstock conversion. However, the processes may be operated without employing a catalyst regenerator.

In a typical process, the alkanol and olefinic feedstream is converted in a catalytic reactor under oligomerization conditions and moderate pressure (ie-100 to 2500 kPa) to produce a predominantly liquid product consisting essentially of $C_5$5+ hydrocarbons rich in gasoline-range mono-olefins and aromatics. The $C_5$ + gasoline product comprises at least 45 weight percent aromatics. The use of fluidized bed catalysis allows excellent control of catalyst activity and temperature which can be used to maintain relatively constant product quality. In addition, it provides excellent flexibility to change operating conditions.

The light aliphatic hydrocarbon conversion process to form aromatics may utilize conversion conditions described in U.S. Patent No. 3,760,024 (Cattanach); 3,845,150 (Yan and Zahner); 4,097,367 (Haag et al.); 4,350,835 (Chester et al.); 4,590,323 (Chu); and 4,629,818 (Burress). Preferred aromatization conditions include temperatures of about 400°C-600°C and pressure from about 100-2000 kpa, absence of hydrogen and a weight hourly space velocity of from 0.5 to 20 WHSV . The C6-C10 aromatic products which are produced are predominantly benzene, toluene and xylene isomers (BTX) with minor amounts of other $C_6$-$C_{10}$ components.

While the instant invention has been described by specific examples and embodiments, the skilled person will appreciate that modification may be made within the scope of the appended claims.

## Claims

1. A continuous process for converting lower aliphatic alkanol and olefinic hydrocarbon to alkyl tertiary-alkyl ethers and C5 + gasoline boiling range hydrocarbons comprising the steps of:
  (a) contacting alkanol and a light olefinic hydrocarbon steam rich in C4 isomeric hydrocarbons, preferably including isobutylene, under iso-olefin etherification conditions in an etherification reaction zone containing acid etherification catalyst;
  (b) separating etherification effluent from step (a) to recover a vapor stream comprising unreacted alkanol and light olefinic hydrocarbon and a liquid product stream containing alkyl tertiary-butyl ether; and
  (c) contacting said unreacted alkanol and light olefinic hydrocarbon vapor stream with acidic, medium pore metallosilicate catalyst under alkanol and hydrocarbon conversion conditions whereby C5 + gasoline boiling range hydrocarbons are produced.

2. A process according to claim 1, wherein said alkanol includes methanol, ethanol, 1-propanol, isopropanol, 1-butanol, 2-butanol and mixtures thereof, and said alkanol is preferably methanol.

3. A process according to claim 1 or 2, wherein the alkanol feedstock comprises dry methanol and wherein the hydrocarbon consists essentially of C4 paraffins and olefins.

4. A process according to claim 1, 2 or 3, wherein the alkyl tertiary-butyl ether produced in step (b) comprises methyl tertiary butyl ether.

5. A process according to claim 1, wherein said light olefinic hydrocarbon stream comprises C4 + olefinic hydrocarbons and said liquid stream contains alkyl tertiary butyl ether and alkyl tertiary amyl ether.

6. A process according to any preceding claim, wherein the step (c) alkanol and hydrocarbon conversion conditions comprise temperature between 400 and 600°C, pressure between 100 and 2000 kPa, and said gasoline boiling range hydrocarbons are rich in C6-C9 aromatic hydrocarbons.

7. A process according to any of claims 1 to 5 wherein in step (c) alkanol and hydrocarbon conversion conditions comprise a temperature of about 425°C, pressure of about 925 kPa, and said gasoline boiling range hydrocarbons are predominantly aromatic hydrocarbons.

8. A process according to any preceding claim, wherein said light olefinic hydrocarbon stream consists essentially of C4 hydrocarbon rich in isobutylene.

9. A process according to claim 8, wherein said light hydrocarbon stream comprises about 80 weight percent mixed butanes and butenes.

10. A process according to any preceding claim, wherein said metallosilicate catalyst comprises acid zeolite having the structure of ZSM-5 and said etherification catalyst comprises acidic zeolite or sulfonated vinyl aromatic resins.

11. A process according to any preceding claim, wherein the C5 + gasoline product comprises at least 45 weight percent aromatics.

12. A process according to any preceding claim, wherein in step (a) the molar ratio of said alkanol to iso-olefin is between substantially 0.7 and 2;

13. A process according to claim 12, wherein the ratio of alkanol to iso-olefin is substantially 1 to 1.

14. A process according to claim 1, wherein said light olefinic hydrocarbon stream comprises C4 + olefinic hydrocarbons and said liquid stream contains alkyl tertiary butyl ether and alkyl tertiary amyl ether.

15. A process according to any preceding claim, wherein the feedstream mixture contains a minor amount of lower alkanol and a major amount of C4-olefin.

16. A process according to claim 15, wherein said feedstream mixture contains at least 80 weight percent mixed butenes.

17. A process according to claim 15 or 16, wherein said feedstream mixture contains methanol in the amount of 0.1 to 5 weight percent, based on butenes.

18. A process according to any preceding claim, comprising the further step of introducing an additional feedstream comprising C2-C3 lower olefins to said reaction zone.

FIG. 1

C5+ GASOLINE

MTBE

OFF-GAS

LPG

METHANOL

C4

FLUID BED REACTOR

REGEN.

10 12 14 15 16 20 22 24 26 30 32 34 36 40 44 45 46 47 48

European
Patent Office

**EUROPEAN SEARCH
REPORT**

Application Number

**EP 90 30 8083**

# DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-4 830 635   (HARANDI et al.)<br>* Claims 1,13,15; column 5, lines 49-68; column 6, lines 13-51 *<br>– – – | 1,2,3,4,5, 8,10,14 | C 10 G 3/00<br>C 10 L 1/02 |
| A | US-A-4 629 818   (BURRESS)<br>* Claims 1,9,12 *<br>– – – – – | 1,6,7,10 | |

|  |  |
|---|---|
|  | TECHNICAL FIELDS SEARCHED (Int. Cl.5) |
|  | C 10 G<br>C 10 L |

The present search report has been drawn up for all claims

| Place of search | Date of completion of search | Examiner |
|---|---|---|
| The Hague | 30 October 90 | DE HERDT O.C.E. |

CATEGORY OF CITED DOCUMENTS

X : particularly relevant if taken alone
Y : particularly relevant if combined with another
 document of the same catagory
A : technological background
O : non-written disclosure
P : intermediate document
T : theory or principle underlying the invention

E : earlier patent document, but published on, or after
 the filing date
D : document cited in the application
L : document cited for other reasons

 

& : member of the same patent family, corresponding
 document